# EUROPEAN PATENT APPLICATION

(11) **EP 2 113 206 A2**
(43) Date of publication of application: **04.11.2009**
(21) Application number: 09251241.7
(22) Date of filing: 01.05.2009
(51) Int. Cl.: A61B 17/06, A61B 19/02, A61F 2/00

(54) **Medical device package**

(30) Priority: 01.05.2008 US 49504 P; 29.04.2009 US 431837
(71) Applicant: Tyco Healthcare Group LP, North Haven, CT 06473 (US)
(72) Inventor: Stopek, Joshua B., Yalesville, CT 06492 (US)
(74) Representative: Soames, Candida Jane

(57) **Abstract**

A package for a medical device, the package capable of sustaining viable cells, wherein the package includes a first container, which is configured to receive a medical device and to sustain at least one viable cell, and a fluid port in communication with the first container for allowing sterile passage of an agent to the medical device and for maintaining cell viability.

## Description

### CROSS-REFERENCE TO RELATED PATENT APPLICATIONS

This application is a continuation-in-part of co-pending U.S. Patent Application Serial No. 11/954,426 filed on December 12, 2007 which is a continuation-in-part of co-pending U.S. Patent Application Serial No. 11/340,912 filed on January 26, 2006, the disclosures of which are herein incorporated by reference in their entireties.

### BACKGROUND

### 1. Technical Field

The present disclosure relates to medical device packaging and, more particularly, to medical device packages capable of sustaining viable cells, including a container for receiving a medical device and at least one viable cell.

### 2. Discussion of Related Art

Tissue engineering and cell transplantation are emerging fields in which few hybrid medical devices, e.g., medical devices combined with bioactive agents such as viable cells, tissue fragments, antibodies and the like, are currently available. While significant efforts have been made relating to the development of biocompatible synthetics and naturally derived biomaterials and the capture of bioactive agents on device or tissue surfaces, little work has been done on developing package materials and package design to make hybrid medical devices available.

Often, the end user, e.g., clinicians and/or researchers, must transfer the bioactive agent and the medical device from separate sterile storage devices to a common sterile culture flask or reactor. Once combined, the cells may be cultured before the medical device/bioactive agent combination is transferred from the sterile culture flask to another containment device for storage during transportation to the implantation site. The risk of damage and contamination of the medical device generally increases each time the medical device and/or the bioactive agent is transferred from one sterile environment to another.

Thus, it would be beneficial to offer an end user a package suitable for receiving a medical device wherein the package can be seeded with viable cells and maintains the viable cells in a constant sterile environment without the need for removal of the device or the cells from the sterile environment prior to implantation or other procedure.

### SUMMARY

The present disclosure relates to a package for a medical device wherein the package is capable of sustaining viable cells. The package includes a first container configured to receive a medical device and to sustain at least one viable cell. The package also includes a fluid port in communication with the first container for allowing sterile passage of an agent to the medical device and for maintaining cell viability.

### BRIEF DESCRIPTION OF THE DRAWINGS

Objects and features of the presently disclosed packages capable of sustaining viable cells will become apparent to those of ordinary skill in the art when descriptions of various embodiments thereof are read with reference to the accompanying drawings, of which:
FIG. 1 is a top view of a package suitable for receiving a medical device and capable of sustaining viable cells in a constant sterile environment in accordance with the present disclosure;
FIG. 2 is a top view of a package suitable for receiving a surgical suture and capable of sustaining viable cells in a constant sterile environment in accordance with the present disclosure; and
FIG. 3 is a top view of a package suitable for receiving a surgical mesh and capable of sustaining viable cells in a constant sterile environment in accordance with the present disclosure.

### DETAILED DESCRIPTION

Hereinafter, embodiments of the presently disclosed packages will be described with reference to the accompanying drawings. Like reference numerals may refer to similar or identical elements throughout the description of the figures. As it is used in this description, "medical device" generally refers to an object which is used for medical purposes, for example, in diagnosis, therapy or surgery. As it is used in this description, "suture" generally refers to a material used surgically to join tissues. Sutures may be constructed from a variety of materials including surgical gut, silk, cotton, polyolefins such as polyglycolic acid, glycolide-lactide copolymer, or a wide variety of polyesters derived from polyglycolic acid, or any material or combination of materials adapted for use to join tissues. As it is used in this description, "medically useful periods of time" generally refers to periods of time beginning when viable cells are added to a package and ending when a medical device and/or the viable cells are withdrawn from the package. Medically useful periods of time may range from seconds to years, wherein the actual time period may depend upon the medical device and/or the type of cell stored in the package. As it is used in this description, "fluid" generally refers to a liquid, gas or both.

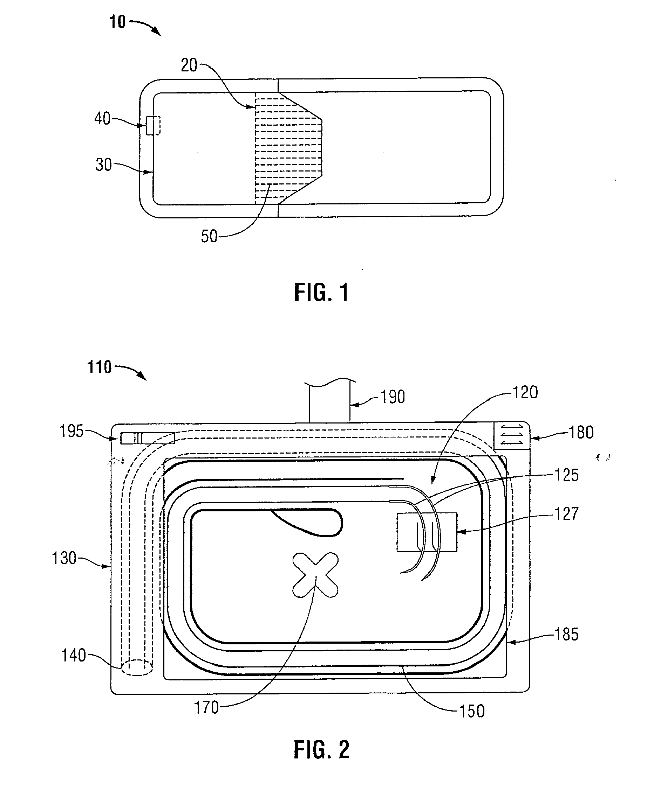

Referring now to FIGS. 1-3, packages according to the present disclosure are shown and include a container (e.g., 30 shown in FIG. 1), which is designed and configured to receive a medical device (e.g., 20 shown in FIG. 1) and also to sustain at least one viable cell (e.g., 50 shown in FIG. 1). The presently disclosed packages further include at least one fluid port (e.g., 140 shown in FIG. 2), which is in communication with the container for allowing sterile passage of an agent to the medical device and for maintaining cell viability. Various medical devices may be received within one or more containers of the presently disclosed packages, including biologically based medical devices and non-biologically based medical devices.

Biologically based medical devices generally include implantable, transplantable or prosthetic devices made or derived from natural tissues. The natural tissues may be derived from any animal source including bovine, porcine, human, shark, etc. Examples of biologically based medical devices include heart valves, bypass grafts, tendons, ligaments, skin grafts, cartilage, bone grafts, vascular grafts, dialysis grafts, aortic grafts, tissue engineered scaffolds, ocular implants, retinal implants, optic nerve implants, corneal implants, organ transplants, biological tissue harvested from the patient (autograft) or donors (allograft) or from a species other than man (xenograft), and any other tissue based devices.

Non-biologically based medical devices generally include implantable, transplantable or prosthetic devices made from any material other than natural unprocessed tissues. Non-biologically based materials that can be used to form the medical devices described herein include, without limitation, a variety of biocompatible materials such as metals, e.g., titanium, stainless steel, and nickel titanium (NiTi), also commonly referred to as nitinol, glass, ceramics, nylons, and polymeric materials. Polymers may include bioabsorbable and non-bioabsorbable materials, such as polystyrene, polycarbonate, polytetrafluoroethylene, polyethylene, polypropylene, polysaccharides, polylactides, polyglycolides, polydioxanones, polytrimethylene carbonate, and combinations thereof.

Some examples of non-biologically based medical devices which may be received within the presently disclosed packages include filamentous materials, e.g., sutures (braided, monofilaments, barbed and combinations thereof), staples, clips, pledgets, buttresses, suture anchors, cables, wires, pacemakers, stents, catheters, inflatable devices, adhesives, sealants, meshes, sternum closures, pins, screws, tacks, rods, plates, adhesion barriers, bioelectronic devices, dental implants, filler materials, surgical tools, buttresses, drug delivery devices, scaffolds, films, foams, synthetic graft materials, cannulas, trocars, and combinations thereof.

Embodiments of the presently disclosed packages include at least one portable container suitable for receiving a medical device and sustaining viable cells for medically useful periods of time. The container may take the shape of any suitable enclosure for storing medical devices manufactured from any suitable material, such as the biologically- and non-biologically-based materials described above. The container (e.g., 130 shown in FIG. 2) is used to maintain a sterile environment capable of sustaining viable cells within the package (e.g., 110 shown in FIG. 2).

In embodiments, the container is a sealed monolithic or one-piece structure. In other embodiments, the container is a made from a plurality of components and can be assembled to form at least one sealed cavity for storing at least one medical device. The components of the container can be assembled using any suitable means for forming a sealable container including, but not limited to, threads, snaps, rivots, adhesives, and the like. As used herein, the terms "sealed" and "sealable" are meant to infer that the ingress of bacteria and other microscopic materials into the container is prevented, without the activity of the fluid port. Some examples of enclosures that may be suitable as containers include, but are not limited to, pouches, plastic retainers, envelopes, foil-packs, and the like. In one embodiment, the container is formed by heat sealing two panels of aluminum foil coated on the interior surfaces thereof with a heat sealable polymeric composition, such that the foil-pack is bonded around the periphery of the container. Other means for sealing the container may be employed as is well known to those skilled in the art. In other embodiments, at least one layer of aluminum foil or polymeric material may be positioned around the outer periphery of a plastic container to form a sealable package. In still other embodiments, more than one container may be formed within the package for receiving one or more medical devices.

The container may be manufactured from any material that is suitable for receiving or storing a medical device. Some examples of materials that may be suitable for receiving or storing a medical device include, but are not limited to, polycarbonate, high-density polyethylene, polyethylene, polypropylene, thermoplastic elastomers, thermosets, thermoplastic resins, poly(ethylene terephthalate), polytetrafluoroethylene, ε-caprolactone, glycolide, 1-lactide, d,1-lactide, d-lactide, meso-lactide, trimethylene carbonate, 4,4-dimethyl-1,3-dioxan-2-one, p-dioxanone, dioxepanone, δ-valerolactone, β-butyrolactone, ε-decalactone, 2,5-diketomorpholine, pivalolactone, α,α-diethylpropiolactone, 6,8-dioxabicyclooctan-7-one, ethylene carbonate, ethylene oxalate, 3-methyl-1,4-dioxane-2,5-dione, 3,3-dimethyl-1,4-dioxane-2,5-dione, polyolefins, polysiloxanes, polyalkylene glycols, polyacrylates, aminoalkyl acrylates, polyvinylalcohols, polyvinylpyrrolidones, polyoxyethylenes, polyacrylamides, poly(2-hydroxy-ethylmethacrylate), polymethacrylamide, dextran, alginic acid, sodium alginate, polysaccharides, gelatin and copolymers, homopolymers, and block copolymers thereof.

The sealed packages described herein may be suitable for use to sustain viable cells in a constant sterile environment for medically useful periods of time. During medically useful periods of time, the viable cells may interact with the medical device in a medically useful manner without removal from the constant sterile environment inside the package. The cells may be allowed to grow on the device, or cultured in the package for collection and analysis, or allowed to interact with the medical device, e.g., to load the medical device with a certain byproduct produced by the viable cells, or, in the case of stem cells, the cells may be allowed to develop into other cells.

In some embodiments, a medical device and viable cells may be sealed within the package during the manufacturing process and may be sterilized, shipped and stored together for extended periods of time before the removal of the medical device and/or the cells from the package. In other embodiments, the medical device may be sealed in the package during the manufacturing process and the viable cells may be added to the package via the fluid port at some time after the package has been sealed. In embodiments, the package may be able to maintain the viability of the cells from about 0.01 seconds to about 5 years, depending on the type of implant and/or medical application.

In addition to the container and the medical device, embodiments of the presently disclosed packages include at least one fluid port (e.g., 140 shown in FIG. 2). The fluid port is designed to permit the passage of at least one agent between the outside of the package or container and the medical device and/or the viable cells, which are contained inside the sealed container. The agent may be in any physical form, e.g., it may be a solid, semi-solid, liquid, gas, and combinations thereof. The fluid port may be sealable, stationary, movable, and combinations thereof. In embodiments, the fluid port may be used to exchange growth medium, cell nutrients, or remove waste product to maintain the viability of the cells. In embodiments, the fluid port may be used to pass viable cells into the package. The presently disclosed medical device packages are also designed and configured to sustain viable cells. In embodiments, the packages suitable for receiving medical devices are able to produce conditions (an environment) which allow the viable cells to grow and be cultured within the confines of the package, thereby eliminating the need to transport cells or devices from one sterile environment to another to form a combination device and, thus, decreasing the likelihood of contamination and damage to the sterile products. Examples of such conditions which will be described later include but are not limited to controlled temperature, pressure, humidity, oxygen and gas levels.

Viable cells generally include any single or multiple cell complex capable of growth or culturing. The cells may be derived from humans or any other animal. The cells may be xenogeneic, allogeneic, autogeneic, isogeneic, homogeneic or any combination thereof. The cells may be genetically modified. Some non-limiting examples include stem cells, red blood cells, white blood cells, cancerous cells, epithelial cells, secretory cells, contracile cells, blood and immune system cells, sensory transducer cells, autonomic neuron cells, glial cells, lens cells, pigment cells, germ cells, nurse cells, basal cells, hematapoetic cells, muscle cells, tendon cells, ligament cells, cardiac cells, islet cells, myocyte cells, and combinations thereof. In embodiments, the package includes a medical device and viable stem cells.

An agent may be selected from any bioactive and/or non-bioactive agent suitable for combination with the medical device and the viable cell. Suitable agents may include, but are not limited to, growth medium, cell nutrients, growth factors, peptides, proteins, enzymes, antibodies, cell receptors, fibronectin, laminin, morphogenic factors, cell matrix proteins, genetic materials, deoxyribonucleic acids, ribonucleic acids, viral vectors, nucleic acids, lymphokines, plasmids, and drugs. Some non-limiting examples of useful drug compounds include antiseptics, anesthetics, muscle relaxants, antihistamines, decongestants, antimicrobial agents, anti-viral agents, anti-fungal agents, antimalarials, amebicides, antituberculosal agents, antiretroviral agents, chemotherapeutics, leprostatics, antiprotazoals, antihelmitics, antibacterial agents, steroids, hematopoietic agents, antiplatelet agents, anticoagulants, coagulants, thrombolytic agents, hemorrheologic agents, hemostatics, plasma expanders, hormones, sex hormones, uterine-active agents, bisphosphonates, antidiabetic agents, glucose-elevating agents, growth hormones, thyroid hormones, inotropic agents, antiarrhythmic agents, calcium channel blockers, vasodilators, sympatholytics, antihyperlipidemic agents, vasopressors, angiotensin antagonists, sclerosing agents, anti-impotence agents, urinary alkanizers, urinary acidifiers, anticholinergics, diuretics, bronchodilators, surfactants, antidepressants, antipsychotics, antianxiety agents, sedatives, hypnotics, barbiturates, antiemetic agents, analgesics, stimulants, anticonvulsants, antiparkinson agents, proton pump inhibitors, H₂-antagonists, antispasmodics, laxatives, antidiarrheals, antiflatulents, digestive enzymes, gallstone solubilizing agents, antihypertensive agents, cholesterol-lowering agents, radiopaque agents, immune globulins, antitoxins, antivenins, immunologic agents, anti-inflammatory agents, antineoplastic agents, alkylating agents, antimetabolites, antimitotic agents, radiopharmaceuticals, vitamins, herbs, trace elements, amino acids, chelating agents, immunomodulatory agents, immunosuppressive agents, diluents, radioactive agents, immuneglobulis, preservatives, colorants, dyes, ultraviolet absorbers, ultraviolet stabilizers, photochromic agents, anti-adhesives, polysaccharides, growth factor antagonists, anti-colonization agents, diagnostic agents, imaging agents, and combinations thereof.

In some embodiments, the agent includes any agent previously described herein combined with a polymeric material. The agent can be combined with a polymer in any suitable manner, such as, for example, by physically admixing, embedding or dispersing the agent in the polymer matrix. In embodiments, the agent is attached directly to a polymer, chemically linked to a polymer through a linker or spacer molecule, directly or indirectly chemically linked to a chemical group attached to the backbone of a polymer and/or electrostatically attached to the polymer or the polymer backbone. The agents can be attached to repeating units of a polymer by covalent bonds, providing for sustained release of the active agent or it may merely reside in the unoccupied spaces present in a polymer. In another embodiment, the agent may form a salt with a polymer or a polymer backbone. In one embodiment, the agent is located in the unoccupied spaces present in a polymer and is present as a homogeneous functional group or it may be incorporated into a salt, micelle, liposome, or heterogeneous aggregate.

In embodiments, the presently disclosed packages may include a variety of devices useful in maintaining cell viability. Some examples include a gas exchange portal, an agitator, a power supply, a temperature element, a thermostat, a humidity control, an observation window and combinations thereof. In certain embodiments, the packages include a gas exchange portal to provide for sterile respiration to the container. The gas exchange portal is in communication with the container and facilitates the introduction and removal of gaseous materials, such as oxygen, nitrogen, and/or carbon dioxide, from the container under sterile conditions to maintain the viability of the cells. In some embodiments, the gas exchange portal may be removable or interchangeable, which may help to improve the capability to maintain the viable cells within the packages for longer periods of time.

In certain embodiments, the packages may also include an observation window, which may be positioned adjacent the container to permit the observation of the medical device positioned in the container. The observation window may be made from any transparent or semi-transparent material, e.g., clear polymeric materials and glass. The observation window allows the user of the package to observe the growth and culturing of the viable cells and/or the medical device disposed inside the container.

In some embodiments, the packages described herein may also include a power source capable of operating electronic controls, such as a thermostat, an agitator, a temperature control or a humidity control. In alternate embodiments, the power source includes one or more batteries, e.g., alkaline batteries, wet cell batteries, dry cell batteries, nickel cadmium batteries, lithium batteries, or NiMH (nickel metal hydride) batteries and the like. In some embodiments, the packages may include a series of solar panels capable of generating solar power. In still other embodiments, the packages may be adapted to be plugged into an electric outlet and the package may be driven by AC or DC power. Various devices such as sensors, controls, agitators, thermostats and the like may be connected to the power source as is known to those skilled in the art. In embodiments, these additional devices may be controlled by a microprocessor which may also be connected to the power supply.

In embodiments where the package includes a power source, controls may be provided for monitoring and/or controlling the environment inside the container. Such controls may enhance the capability to maintain viable cells within the packages. Controls that may be provided include, but are not limited to, temperature controls which may heat or cool the container to a selected temperature set point or temperature range, humidity controls which may adjust the moisture level inside the container to a set humidity level (or range), and an agitator control, which may allow the contents inside the containers to be stirred, pulsed or mixed in a variety of ways, at a variety of speeds and for various time periods.

In embodiments, the agitator may be a magnetic capsule or rod placed inside the container. The magnetic capsule or rod may be controlled by a separate device outside of the package, such as a magnetic stirring device. The package containing the magnetic rod may be placed on the stirring device and, through the use of magnetic forces, the agitator positioned inside the package could be controlled to spin or mix the contents of the package. In other embodiments, the agitator may be in the form of a paddle wheel or fan which may be connectable to the power source via a control switch on the package. In such embodiments, the control switch could be activated to allow the agitator to spin inside the package. In still other embodiments, the package may be designed and configured to be received by a separate rotational device whereby the entire package may be spun inside the rotational device.

Turning now to FIG. 1, a package according to an embodiment of the present disclosure is depicted generally as 10 and includes a container 30 configured to receive a medical device 20 and to sustain at least one viable cell in a constant sterile environment inside the package 10. Package 10 is provided with a fluid port 40, which may be positioned along any side, edge or corner of the container 30. In embodiments wherein the package 10 includes more than one container 30, the fluid port 40 may be positioned along any side, edge or corner of any of the cavities included in the package 10. Viable cells 50 are positioned on at least a portion of the medical device 20 which is positioned with the container 30.

Fluid port 40 is shown as an injectable-hub which is designed to remain sealed by self-sealing action to ensure no fluid medium can escape and also so no pathogens can breach the container. Fluid port 40 can be composed of a rubber or thermoplastic material, such as known to be used in sealing sterile vials, intravenous bags, catheters, drug ampules or blood bags. Alternatively, the fluid port 40 may be composed of hydrophobic, hydrophilic or a combination of hydrophobic and hydrophilic materials. The size, shape and dimensions of the fluid port 40 may be varied from an exemplary configuration depicted in FIG. 1.

In one embodiment, fluid port 40 may be a hub designed in such a way that only a particular syringe can mate with fluid port 40 thereby creating a lock and key type of hub to promote only specific use of fluid port 40. This type of hub may help to provide safety to the user of fluid port 40 because the hub does not necessarily require the use of a needle. In addition, the lock and key type of hub may be used by patients and medical staff for only certain medications and dosages of those medications, thereby tending to reduce the likelihood of administering the wrong agent or the wrong dosage of the intended agent.

Referring to FIG. 2, a package 110 is shown and includes a container 130 and a fluid port 140, wherein the fluid port 140 penetrates into the container 130. Container 130 also includes a medical device 120, coated with viable cells (not shown), and an agitator 170 to facilitate a mixing action. In embodiments, package 110 includes a gas exchange portal 180 and a power source 190, e.g., prongs capable of being plugged into an electrical outlet. In embodiments, the agitator 170 is controlled by a control element 195 which is connected to the power supply 190. In FIG. 2, the medical device 120 is composed of two sutures 150, wherein each suture 150 connected to a needle 125 which is stored in a needle park 127 positioned within the container 130. Container 130 may be configured to receive any number and type of medical device. Package 110 may include an observation window 185 so at least a portion of the inside of the container 130 and/or at least a portion of the medical device 120 is visible from outside the package 110.

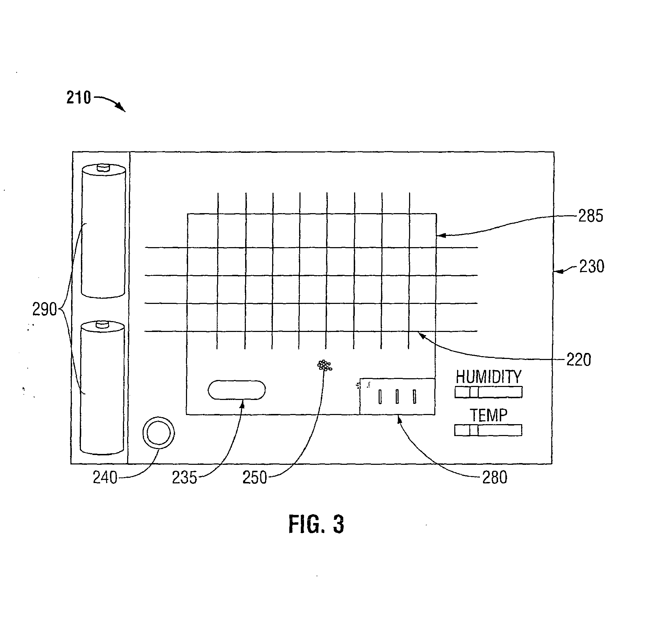

Turning to FIG. 3, a package 210 is shown and includes a container 230, a medical device 220, a fluid port 240 and viable cells 250. Fluid port 240 may be sealable and/or movable. In addition, package 210 includes a power supply 290, a gas exchange portal 280 and an observation window 285. Power supply 290 may be powered by alkaline or rechargeable batteries. In an embodiment illustrated in FIG. 3, the medical device 220 is shown as a surgical mesh and the viable cells 250 are shown positioned in the container 230 separate from the medical device 220. The size, shape, dimensions and positioning of the medical device 220, the fluid port 240, and the viable cells 250 may be varied from an exemplary configuration depicted in FIG. 3.

Package 210 may include a second container 235. In embodiments, the medical device 220 and the viable cells 250 are separately positioned in the first and second containers, 230 and 235. Second container 235 may be adapted to communicate with the fluid port 240 and/or may be adapted to include a second fluid port (not shown) for allowing the sterile passage of an agent to a medical device and/or for maintaining cell viability.

Although embodiments of the present disclosure have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.

## Claims

1. A package for a medical device, the package capable of sustaining viable cells, the package comprising:
a first container configured to receive a medical device and to sustain at least one viable cell;
a fluid port in communication with the first container for allowing sterile passage of an agent to the medical device and for maintaining cell viability.

2. The package of claim 1, wherein the at least one viable cell is sustained in a constant sterile environment inside the package.

3. The package of claim 1, wherein the at least one viable cell is sustained in a constant sterile environment without the need for removal of the medical device or the at least one viable cell from the sterile environment prior to implantation.

4. The package of claim 1, wherein the medical device includes a biologically derived material.

5. The package of claim 4, wherein the biologically derived material is selected from the group consisting of heart valves, bypass grafts, tendons, ligaments, skin grafts, cartilage, bone grafts, vascular grafts, dialysis grafts, aortic grafts, tissue scaffolds, ocular implants, retinal implants, optic nerve implants, corneal implants, organ transplants, autografts, allografts, and xenografts.

6. The package of claim 1, wherein the medical device is selected from the group consisting of sutures, staples, clips, pledgets, buttresses, suture anchors, cables, wires, pacemakers, stents, catheters, inflatable devices, adhesives, sealants, meshes, sternum closures, pins, screws, tacks, rods, plates, adhesion barriers, bioelectronic devices, dental implants, surgical tools, buttresses, drug delivery devices, scaffolds, films, foams, synthetic graft materials, cannulas, trocars, and combinations thereof.

7. The package of claim 1, wherein the medical device includes a suture.

8. The package of claim 1, wherein the at least one viable cell is selected from the group consisting of stem cells, red blood cells, white blood cells, cancerous cells, epithelial cells, secretory cells, contracile cells, blood and immune system cells, sensory transducer cells, autonomic neuron cells, glial cells, lens cells, pigment cells, germ cells, nurse cells, basal cells, hematapoetic cells, muscle cells, cartilage cells, tendon cells, ligament cells, cardiac cells, and combinations thereof.

9. The package of claim 1, where the at least one viable cell is a stem cell.

10. The package of claim 1, further comprising a second container.

11. The package of claim 10, wherein the medical device and the at least one viable cell are separately positioned in the first and second containers.

12. The package of claim 1, wherein the agent is growth medium, cell nutrients, and combinations thereof.

13. The package of claim 1, wherein the agent is selected from the group consisting of growth medium, viable cells, growth factors, peptides, proteins, enzymes, antibodies, cell receptors, fibronectin, laminin, morphogenic factors, cell matrix proteins, deoxyribonucleic acids, ribonucleic acids, modified genetic materials, viral vectors, nucleic acids, lymphokines, plasmids, and drugs.

14. The package of claim 13, wherein the drug is selected from the group consisting of antiseptics, anesthetics, muscle relaxants, antihistamines, decongestants, antimicrobial agents, anti-viral agents, anti-fungal agents, antimalarials, amebicides, antituberculosal agents, antiretroviral agents, chemotherapeutics, leprostatics, antiprotazoals, antihelmitics, antibacterial agents, steroids, hematopoietic agents, antiplatelet agents, anticoagulants, coagulants, thrombolytic agents, hemorrheologic agents, hemostatics, plasma expanders, hormones, sex hormones, uterine-active agents, bisphosphonates, antidiabetic agents, glucose-elevating agents, growth hormones, thyroid hormones, inotropic agents, antiarrhythmic agents, calcium channel blockers, vasodilators, sympatholytics, antihyperlipidemic agents, vasopressors, angiotensin antagonists, sclerosing agents, anti-impotence agents, urinary alkanizers, urinary acidifiers, anticholinergics, diuretics, bronchodilators, surfactants, antidepressants, antipsychotics, antianxiety agents, sedatives, hypnotics, barbiturates, antiemetic agents, analgesics, stimulants, anticonvulsants, antiparkinson agents, proton pump inhibitors, H₂-antagonists, antispasmodics, laxatives, antidiarrheals, antiflatulents, digestive enzymes, gallstone solubilizing agents, antihypertensive agents, cholesterol-lowering agents, radiopaque agents, immune globulins, monoclonal antibodies, antibodies, antitoxins, antivenins, immunologic agents, anti-inflammatory agents, antineoplastic agents, alkylating agents, antimetabolites, antimitotic agents, radiopharmaceuticals, vitamins, herbs, trace elements, amino acids, enzymes, chelating agents, immunomodulatory agents, immunosuppressive agents, and combinations thereof.

15. The package of claim 1, further comprising a temperature controller to heat or cool the package to a selected temperature set point or temperature range; or the package of claim 1, further comprising a humidity controller to control a moisture level inside the package to a set humidity level or a range of humidity; or the package of claim 1, further comprising an agitator to facilitate a mixing action; or the package of claim 1, further comprising a window to allow visual observation of at least a portion of the medical device or the at least one cell; or the package of claim 1, further comprising a gas exchange portal; preferably wherein the gas exchange portal is removable from the package.
